# EUROPEAN PATENT APPLICATION

(11) **EP 2 742 963 A1**
(43) Date of publication of application: **18.06.2014**
(21) Application number: 12197387.9
(22) Date of filing: 17.12.2012
(51) Int. Cl.: A61M 5/32

(54) **Drug delivery device**

(71) Applicant: Sanofi-Aventis Deutschland GmbH, 65929 Frankfurt am Main (DE)
(72) Inventor: The designation of the inventor has not yet been filed

(57) **Abstract**

The invention relates to a drug delivery device (1) for dispensing a dose of a drug, comprising:
- a support body (2) having a first end defining a first opening and a second end defining a second opening,
- a syringe barrel (5) sealed by a stopper (7) slidably disposed therein along an axial direction and adapted to have an integrated needle (6) or to be coupled with a needle assembly comprising a needle (6), wherein the syringe barrel (5) is positioned within the support body (2),
- a protective needle boot (11) arrangeable over the needle (6) on the syringe barrel (5),
- a needle shield (4) adapted to be coupled to one of the ends of the support body (2) and slidable with respect to the support body for covering or exposing the needle (6);
- a locking collar (12) positioned in the support body (2), the locking collar (12) comprising a blocking protrusion (12.1) for obstructing movement of the needle shield (4) relative to the support body (2).

## Description

### Technical Field

The present invention relates to drug delivery device with inherent needle safety comprising a pre-filled syringe.

### Background of the Invention

Pre-filled syringes that are filled with a selected dosage of a medicament are well known injection devices for administering the medicament to a patient. Drug delivery devices comprising a needle shield for covering a needle of a pre-filled syringe before and after use are also well known. Typically, the needle shield is either manually moved or moved by the action of a relaxing spring to surround the needle.

A different type of drug delivery device known in the state of the art solves the object of providing needle safety by arranging the pre-filled syringe movable relative to a body, whereas the pre-filled syringe is retracted into the body after the injection.

### Summary of the Invention

It is an object of the present invention to provide an improved drug delivery device.

The object is achieved by a drug delivery device according to claim 1.

Preferred embodiments of the invention are given in the dependent claims.

In the context of this specification, the terms distal and proximal are defined from the point of view of a person performing an injection. Consequently, a distal direction refers to a direction pointing towards the body of a patient receiving an injection and a distal end defines an end of an element that is directed towards the body of the patient. Respectively, the proximal end of an element or the proximal direction is directed away from the body of the patient receiving the injection and opposite to the distal end or distal direction.

According to the invention a drug delivery device for dispensing a dose of a drug, comprises:
- a support body having a first end defining a first opening and a second end defining a second opening,
- a syringe barrel sealed by a stopper slidably disposed therein along an axial direction and adapted to have an integrated needle or to be coupled with a needle assembly comprising a needle, wherein the syringe barrel is positioned within the support body,
- a protective needle boot arrangeable over the needle on the syringe barrel,
- a needle shield adapted to be coupled to one of the ends of the support body and slidable with respect to the support body for covering or exposing the needle;
- a locking collar positioned in the support body, the locking collar comprising a blocking protrusion for obstructing movement of the needle shield relative to the support body.

This avoids premature exposure of the needle in case a user holds the needle shield when pulling the protective needle boot off the syringe barrel in the distal direction prior to an injection. Thus, pre-injection needle safety is improved and users scared of needles are prevented from seeing the needle.

In an exemplary embodiment the blocking protrusion extends radially outwards through an aperture in the support body.

In an exemplary embodiment a boot remover extends through a central opening in the needle shield and is arranged for removing the protective needle boot. This allows for using a conventional protective needle boot such as a rubber needle boot or rigid needle boot which does not protrude beyond the needle shield of the drug delivery device prior to an injection and can hence not be accessed by the user.

The boot remover may be arranged to engage the needle boot by friction and/or by at least one barb engaging behind a shoulder or within a recess on the protective needle boot.

Likewise the boot remover may be integrally shaped with the protective needle boot.

In an exemplary embodiment the locking collar is substantially ring shaped.

An internal diameter of the locking collar may correspond with an external diameter of the protective needle boot and/or of the boot remover, wherein in an initial state a proximal end of the protective needle boot and/or boot remover is located within the locking collar preventing inward deflection of the blocking protrusion. Thus, the needle shield remains prevented from moving in the proximal direction relative to the support body until the protective needle boot has been at least partially removed from the syringe barrel.

In an exemplary embodiment a distal extension is arranged on the locking collar inwardly from the blocking protrusion for axially overlapping the locking collar and the protective needle boot in the initial state. This allows for keeping the blocking protrusion prevented from being inwardly deflected until the protective needle boot has travelled sufficiently during removal to expire the overlap before the blocking protrusion is allowed to be inwardly deflected for allowing translation of the needle shield in the proximal direction relative to the support body.

In an exemplary embodiment a ramped surface on the blocking protrusion is arranged to be engaged by the proximal end of the needle shield for radially inwardly deflecting the blocking protrusion so as to remove the obstruction preventing movement of the needle shield with respect to the support body in a proximal direction. Thus the blocking protrusion is deflected by movement of the needle shield, e.g. when the needle shield is being pressed against an injection site, so that no further user steps are required.

The locking collar may comprise or consist of a flexible plastic material, elastomer, rubber, etc.

In an exemplary embodiment a needle shield spring is arranged for biasing the needle shield in a distal direction against the support body. Thus the needle shield remains extended prior to application to the injection site and returns to its extended position when removed from the injection site.

The protective needle boot is held on the syringe barrel by friction. A length of the distal extension on the locking collar may be selected to prevent radial inward deflection of the blocking protrusion until the protective needle boot has been moved to such an extent that a remaining friction force between the protective needle boot and the syringe barrel is reduced to an amount smaller than a force of the needle shield spring. At this point the force required to pull the protective needle boot has significantly lowered due to the reduced contact surface and hence reduced friction between the protective needle boot and the syringe barrel such that the remaining resistant force required to prevent movement of the needle shield relative to the support body is provided by the needle shield spring. This increases the reliability of removing the protective needle boot without exposing the needle.

In an exemplary embodiment an outer body is coupled to the other end of the support body and slidably disposed thereon. The outer body may be grabbed by the user for applying the drug delivery device against the injection site.

A plunger rod may be coupled to the stopper, wherein a proximal portion of the plunger rod is adapted to be coupled to the outer body. This allows for displacing the drug from the cavity within the syringe barrel by applying a force to the outer body.

A detent mechanism may be arranged to constrain movement of the outer body relative to the support body. On application of the drug delivery device against the injection site the needle shield moves thus prior to the outer body relative to the support body thus preventing a so called wet injection with drug leaking out of the tip of the needle before the needle reaches an insertion depth.

In an exemplary embodiment the needle shield is slidably disposed on the support body.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a perspective view of a drug delivery device comprising a support body, an outer body and a needle shield in an initial state,
- Figure 2: is a longitudinal section of the drug delivery device in the initial state without the outer body,
- Figure 3: is a perspective view of a locking collar, and
- Figure 4: is a longitudinal section of the drug delivery device after removal of a protective needle boot.

Corresponding parts are marked with the same reference symbols in all figures.

### Detailed Description of Preferred Embodiments

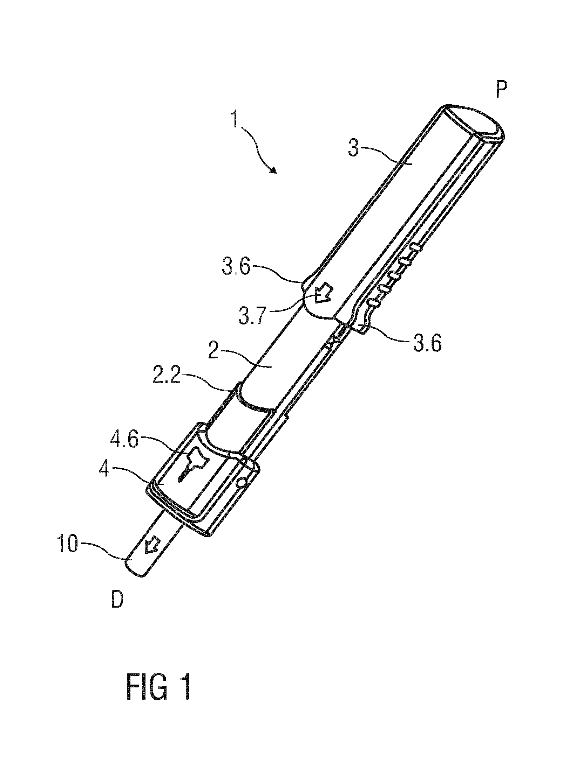

Figure 1 is a perspective view of a drug delivery device 1 comprising a support body 2, an outer body 3 and a needle shield 4, all of them having an essentially tubular shape.

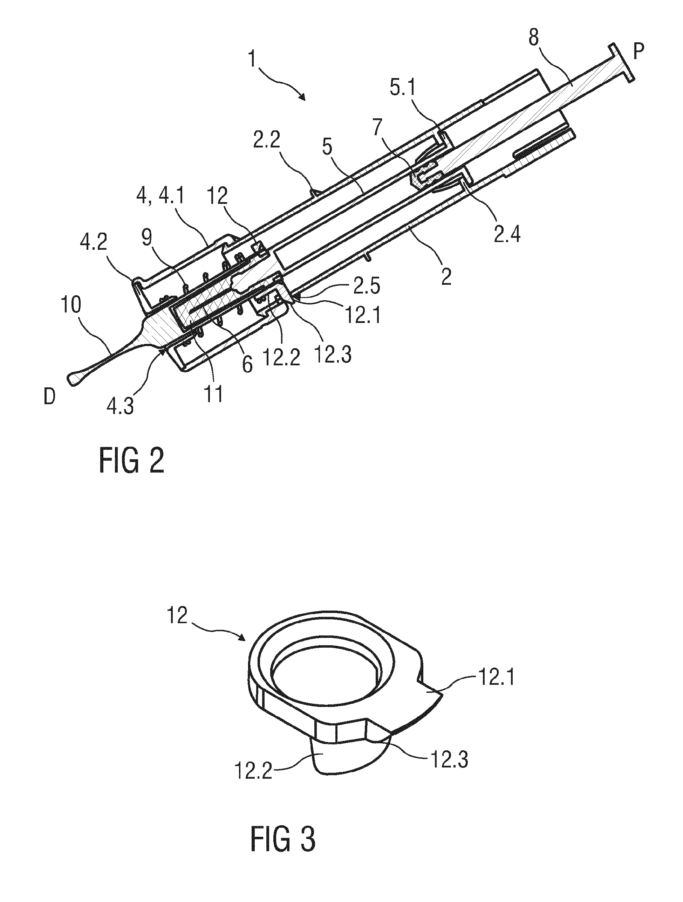

Figure 2 is a longitudinal section of the drug delivery device 1 without the outer body 3. A proximal end of the support body 2 is telescoped in the outer body 3 and a distal end of the support body 2 is telescoped in the needle shield 4 such that the needle shield 4 is slidable between a first position and a second position with respect to the support body 2. A syringe barrel 5 is slidably arranged within the support body 2, the syringe barrel 5 defining an inner cavity for a dose of a drug. A hollow injection needle 6 is arranged on a distal end of the syringe barrel 5. A stopper 7 is slidably arranged within the syringe barrel 5 for sealing its proximal end and for displacing the drug from the syringe barrel 5 through the injection needle 6. A plunger rod 8 is arranged within the outer body 2 for engaging the stopper 7. The plunger rod 8 may be screwed or snapped into the stopper 7. A needle shield spring 9 is arranged for biasing the needle shield 4 in the distal direction D towards the first position against the support body 2.

The outer body 3 is movable in a distal direction D and in a proximal direction P with respect to the support body 2.

The syringe barrel 5 is inserted into the support body 2. A proximal barrel collar 5.1, sometimes referred to as a finger flange, on the syringe barrel 5 serves for attaching the syringe barrel 5 to the support body 2 by distally abutting an internal rib 2.4 in the support body 2 so that the syringe barrel 5 is fixed towards the distal direction D in its axial position with respect to the support body 2.

The support body 2 comprises an indicator ring 2.2 in the shape of radially outwards protruding and essentially circumferential rib. The indicator ring 2.2 may cover part of or the whole circumference of the support body 2. The indicator ring 2.2 is arranged to interact with the needle shield 4 so as to limit distal movement of the support body 2 with respect to the needle shield 4. Furthermore, the support body 2 comprises a locking mechanism and linear guide rails that run in an axial direction for providing a staged movement between the support body 2, the outer body 3 and the needle shield 4.

The needle shield 4 comprises a tubular body section 4.1 and a cap part 4.2 with a central opening 4.3. The cap part 4.2 is engaged to the body section 4.1 by a snap fit (not illustrated) thus preventing relative axial movement. A protective needle boot 11 may be positioned over the needle 6 on the syringe barrel 5 in and/or through the central opening 4.3 of the cap part 4.2 before and after an injection. The cap part 4.2 is rotationally locked to the body section 4.1. This may be achieved by the cap part 4.2 having a non-circular, e.g. elliptical cross section engaging in a corresponding opening in the body section 4.1.

A boot remover 10 is arranged for removing the protective needle boot 11 prior to an injection. The boot remover 10 may be arranged to engage the needle boot 11 by friction or by means of barbs. The boot remover 10 comprises a handle extending from the opening 4.3 for facilitating boot removal. A direction indicator 10.1 may be provided on the boot remover 10 for indicating the direction in which the user has to move the boot remover 10 for removing the protective needle boot. Likewise the boot remover 10 may be arranged as a handle integrally shaped with the protective needle boot 11.

A locking collar 12 is internally arranged in the support body 2 and may be made from a flexible plastic material. Figure 3 is a perspective view of the locking collar 12. The locking collar 12 is substantially ring-shaped and comprises a blocking protrusion 12.1 protruding radially from the locking collar 12. The blocking protrusion 12.1 extends radially outwards through a lateral aperture 2.5 in the support body 2 obstructing movement of the support body 2 into the needle shield 4. An internal diameter of the locking collar 12 corresponds with an external diameter of the protective needle boot 11 or of the boot remover 10. In an initial configuration a proximal end of the protective needle boot 11 or boot remover 10 is located within the locking collar 12 preventing inward deflection of the blocking protrusion 12.1 such that the needle shield 4 cannot be moved in the proximal direction P with respect to the support body 2. A distal extension 12.2 on the locking collar inwardly from the blocking protrusion 12.1 ensures a sufficient overlap of the locking collar 12 and the protective needle boot 11. A ramped surface 12.3 on the locking collar 12 is arranged to be engaged by the proximal end of the needle shield 4 for radially inwardly deflecting the blocking protrusion 12.1 so as to remove the obstruction preventing movement of the needle shield 4 with respect to the support body 2 in the proximal direction P.

Figures 1 and 2 show the drug delivery device 1 prior to an injection. The outer body 3 is fully extended in the proximal direction P from the support body 2. The needle shield 4 is in the first position fully extended from the support body 2 in the distal direction D. The injection needle 6 is in a retracted position within the needle shield 4. The needle shield spring 9 is relaxed.

A user may grab the outer body 3 or the support body 2 or the needle shield 4 and pull the boot remover 10 in the distal direction D for removing the protective needle boot 11.

If the user holds the outer body 3 or the support body 2 the boot remover 10 and the protective needle boot 11 will be pulled out of the central opening 4.3 of the needle shield 4.

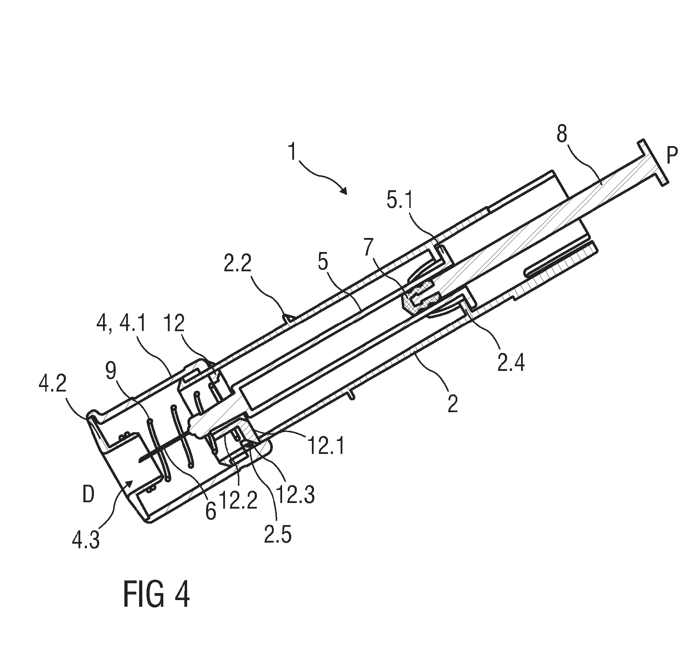

If the user holds the needle shield 4 while pulling the boot remover 10 friction between the protective needle boot 11 and the syringe barrel 5 will tend to pull the syringe barrel 5 and hence the support body 2 in the distal direction D relative to the needle shield 4 which would result in premature exposure of the needle 6. However, due to the locking collar 12 and its blocking protrusion 12.1 the support body 2 cannot be moved in the distal direction D with respect to the needle shield 4. Hence, as the user pulls the boot remover 10 and the protective needle boot 11 against the needle shield 4 the syringe barrel 5 and the support body 2 remain in place. As the force exerted by the user when pulling the boot remover 10 exceeds a friction force between the protective needle boot 11 and the syringe barrel 5 the boot remover 10 and protective needle boot 11 move in the distal direction D relative to the syringe barrel 5 and the locking collar 12. During this movement the overlap between the boot remover 10 and the distal extension 12.2 on the locking collar 12 expires such that the blocking protrusion 12.1 is no longer prevented from being radially inwardly deflected whilst the position of the locking collar 12 in the axial direction relative to the support body 2 is retained. At this point the force required to pull the protective needle boot 11 has significantly lowered due to the reduced contact surface between the protective needle boot 11 and the syringe barrel 5 such that the remaining resistant force required to prevent movement of the needle shield 4 relative to the support body 2 is provided by the needle shield spring 9. The protective needle boot 11 is thus removed without exposing the needle 6 as illustrated in figure 4.

The user may now push the distal end of the needle shield 4 against an injection site, e.g. a patient's skin. The force from the user's hand is resolved through the outer body 3, the support body 2, into the needle shield 4. As the user applies a sufficiently high force the needle shield 4 is moved in the proximal direction P towards the second position with respect to the support body 2 and all other parts of the drug delivery device 1 thereby also compressing the needle shield spring 9. During this movement of the needle shield 4 a proximal end of the needle shield 4 engages the ramped surface 12.3 on the blocking protrusion 12.1 of the locking collar 12 thus radially inwardly deflecting the blocking protrusion 12.1 so that the blocking protrusion 12.1 no longer obstructs the movement of the needle shield 4 relative to the support body 2.

On application of the drug delivery device 1 against the patient's skin the needle shield 4 moves prior to the outer body 3 relative to the support body 2 due to a locking mechanism or detent (not illustrated) between the support body 2 and the outer body 3. This movement is opposed by the friction force of the injection needle 6 when penetrating the skin. In order to avoid a so called wet injection with drug leaking out of the tip of the needle during needle insertion before reaching the insertion depth the friction force of the needle 6 must be less than the counteracting force of the stopper 7 due to friction between the stopper 7 and the inner wall of the syringe 5 and due to the hydrostatic resistance of the drug to be displaced through the hollow needle 6, which depends on the inner diameter of the needle 6 and the viscosity of the drug. The needle insertion depth is defined by the needle shield 4 abutting the indicator ring 2.2. The mating surfaces of the indicator ring 2.2 and the needle shield 4 visually and haptically indicate to the user that they are intended to be pushed completely together in order to correctly apply the drug delivery device 1.

Once insertion depth has been reached, further application of force onto the outer body 3 overcomes the detent so that the outer body 3 decouples from the support body 2 and moves relative to the support body 2 in the distal direction D thus also moving the stopper 7 within the syringe barrel 5 so that the drug is displaced from the cavity through the injection needle 6. Near the end of the injection the stopper 7 bottoms out in the syringe barrel 5. At the same time the outer body 3 abuts the indicator ring 2.2. The mating surfaces of the indicator ring 2.2 and the outer body 3 visually and haptically indicate to the user that they are intended to be pushed completely together in order to correctly apply the drug delivery device 1.

If the user removes the drug delivery device 1 from the injection site the needle shield 4 is no longer pushed against the skin and is hence extended in the distal direction D relative to the other components of the drug delivery device 1 by the needle shield spring 9 such that the injection needle 6 arrives fully inside the needle shield 4. A locking mechanism may be provided between the needle shield 4 and the support body 2 for preventing the needle shield 4 from being retracted once more.

The end of the outer body 3 coupled to the support body 2 may comprise a radially outwards directed flange 3.6 thus improving ergonomics when applying the drug delivery device 1.

In an exemplary embodiment a direction indicator 3.7 designed as a profiled cavity or concavity in the surface of the outer body 3 is arranged in the range of the flange 3.6.

The needle shield 4 may likewise comprise a direction indicator 4.6.

A label retaining recess may be arranged in the outer body 3 for receiving a label which may be customized to the drug to be delivered and/or to the provider of the drug.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 Exendin-4(1-39),
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39),
   wherein the group -Lys6-NH2 may be bound to the C-terminus of the Exendin-4 derivative;
or an Exendin-4 derivative of the sequence
   des Pro36 Exendin-4(1-39)-Lys6-NH2 (AVE0010),
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(02)25, Asp28] Exendin-4(1-39)-Lys6-NH2,
   H-des Asp28 Pro36, Pro37, Pro38 [Trp(02)25] Exendin-4(1-39)-NH2,
   H-(Lys)6-des Pro36, Pro37, Pro38 [Trp(02)25, Asp28] Exendin-4(1-39)-NH2,
   H-Asn-(Glu)5-des Pro36, Pro37, Pro38 [Trp(02)25, Asp28] Exendin-4(1-39)-NH2,
   des Pro36, Pro37, Pro38 [Trp(02)25, Asp28] Exendin-4(1-39)-(Lys)6-NH2,
   H-(Lys)6-des Pro36, Pro37, Pro38 [Trp(02)25, Asp28] Exendin-4(1-39)-(Lys)6-NH2,
   H-Asn-(Glu)5-des Pro36, Pro37, Pro38 [Trp(02)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(02)25, Asp28] Exendin-4(1-39)-Lys6-NH2,
   H-des Asp28 Pro36, Pro37, Pro38 [Met(O)14, Trp(02)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(02)25, Asp28] Exendin-4(1-39)-NH2,
   des Pro36, Pro37, Pro38 [Met(O)14, Trp(02)25, Asp28] Exendin-4(1-39)-(Lys)6-NH2,
   H-(Lys)6-des Pro36, Pro37, Pro38 [Met(O)14, Trp(02)25, Asp28] Exendin-4(S1-39)-(Lys)6-NH2,
   H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: drug delivery device
- 2: support body
- 2.2: indicator ring
- 2.4: internal rib
- 2.5: aperture
- 3: outer body
- 3.6: flange
- 3.7: direction indicator
- 4: needle shield
- 4.1: body section
- 4.2: cap part
- 4.3: central opening
- 4.6: direction indicator
- 5: syringe barrel
- 5.1: barrel collar
- 6: needle
- 7: stopper
- 8: plunger rod
- 9: needle shield spring
- 10: boot remover
- 10.1: direction indicator
- 11: protective needle boot
- 12: locking collar
- 12.1: blocking protrusion
- 12.2: distal extension
- 12.3: ramped surface
- D: distal direction
- P: proximal direction

## Claims

1. Drug delivery device (1) for dispensing a dose of a drug, comprising:
- a support body (2) having a first end defining a first opening and a second end defining a second opening,
- a syringe barrel (5) sealed by a stopper (7) slidably disposed therein along an axial direction and adapted to have an integrated needle (6) or to be coupled with a needle assembly comprising a needle (6), wherein the syringe barrel (5) is positioned within the support body (2),
- a protective needle boot (11) arrangeable over the needle (6) on the syringe barrel (5),
- a needle shield (4) adapted to be coupled to one of the ends of the support body (2) and slidably with respect to the support body (2) for covering or exposing the needle (6);
- a locking collar (12) positioned in the support body (2), the locking collar (12) comprising a blocking protrusion (12.1) for obstructing movement of the needle shield (4) relative to the support body (2).

2. Drug delivery device (1) according to claim 1, wherein a boot remover (10) extending through a central opening (4.3) in the needle shield (4) is arranged for removing the protective needle boot (11).

3. Drug delivery device (1) according to claim 2, wherein the boot remover (10) is arranged to engage the needle boot (11) by friction or by at least one barb.

4. Drug delivery device (1) according to claim 2, wherein the boot remover (10) is integrally shaped with the protective needle boot (11).

5. Drug delivery device (1) according to one of the preceding claims, wherein the blocking protrusion (12.1) extends radially outwards through an aperture (2.5) in the support body (2).

6. Drug delivery device (1) according to one of the preceding claims, wherein the locking collar (12) is substantially ring shaped.

7. Drug delivery device (1) according to claim 6, wherein an internal diameter of the locking collar (12) corresponds with an external diameter of the protective needle boot (11) and/or of the boot remover (10), wherein in an initial state a proximal end of the protective needle boot (11) and/or boot remover (10) is located within the locking collar (12) preventing inward deflection of the blocking protrusion (12.1).

8. Drug delivery device (1) according to claim 7, wherein a distal extension (12.2) is arranged on the locking collar (12) inwardly from the blocking protrusion (12.1) for axially overlapping the locking collar (12) and the protective needle boot (11) in the initial state.

9. Drug delivery device (1) according to one of the preceding claims, wherein a ramped surface (12.3) on the blocking protrusion (12.1) is arranged to be engaged by the proximal end of the needle shield (4) for radially inwardly deflecting the blocking protrusion (12.1) so as to remove the obstruction preventing movement of the needle shield (4) with respect to the support body (2) in a proximal direction (P).

10. Drug delivery device (1) according to one of the preceding claims, wherein the locking collar (12) comprises a flexible plastic material.

11. Drug delivery device (1) according to one of the preceding claims, wherein a needle shield spring (9) is arranged for biasing the needle shield (4) in a distal direction (D) against the support body (2).

12. Drug delivery device (1) according to claim 11, wherein a length of the distal extension (12.2) on the locking collar (12) is selected to prevent radial inward deflection of the blocking protrusion (12.1) until the protective needle boot (11) has been moved to such an extent that a remaining friction force between the protective needle boot (11) and the syringe barrel (5) is smaller than a force of the needle shield spring (9).

13. Drug delivery device (1) according to one of the preceding claims, wherein an outer body (3) is coupled to the other end of the support body (2) and slidably disposed thereon.

14. Drug delivery device (1) according to claim 13, wherein a plunger rod (8) is coupled to the stopper (7), wherein a proximal portion of the plunger rod (8) is adapted to be coupled to the outer body (3).

15. Drug delivery device (1) according to one of the preceding claims, wherein the needle shield (4) is slidably disposed on the support body (2).
